# EUROPEAN PATENT APPLICATION

(11) **EP 3 719 057 A1**
(43) Date of publication of application: **07.10.2020**
(21) Application number: 19167094.2
(22) Date of filing: 03.04.2019
(51) Int. Cl.: C08H 1/06, C08L 89/06, B29C 64/00, C09D 11/04, C07K 14/78

(54) **A GELATIN AND USES THEREOF**

(71) Applicant: Tessenderlo Group NV, 1050 Brussel (BE)
(72) Inventor: CONDI DE GODOI, Fernanda, 3001 Heverlee (BE); DIERCKX, Stephan R.M., 1910 Berg (BE); QUANTEN, Erwin Theo Maria, 3020 Herent (BE); VANDENDRIESSCHE, Jan Maurits August, 1050 Brussel (BE)
(74) Representative: Brantsandpatents bvba

(57) **Abstract**

The present invention relates to a gelatin having improved properties, in particular improved dispensing properties, more particularly improved printing properties. The present invention further relates to a construct produced with the gelatin of the present invention and to a process to produce said construct. Further the present invention relates to the use of the gelatin of the present invention to solve existing problems encountered with dispensing systems, in particular with 3D printing and more particularly for applications in the medical field. The gelatin of the present invention is particularly suitable for bio-printing in the medical field and may also be used in food applications.

## Description

### TECHNICAL FIELD

The present invention relates to a gelatin having improved properties, in particular improved dispensing properties, more particularly improved printing properties. The present invention further relates to a construct produced with the gelatin of the present invention and to a process to produce said construct. Further the present invention relates to the use of the gelatin of the present invention to solve existing problems encountered with dispensing systems, in particular with 3D printing and more particularly for applications in the medical field. The gelatin of the present invention is particularly suitable for bio-printing in the medical field and may also be used in food applications.

### BACKGROUND

Manufacturing technologies in the medical field and also in the food industry, are in constant evolution. Dispensing techniques in particular offer some advantages compared to standard production technologies, particularly in the medical field. Said advantages can be speed of manufacturing, customization and the like. One very well-known dispensing technique is 3D printing. In particular, in the field of medical research, bioprinting is a powerful tool used for tissue engineering applications due to its capability of directly dispensing biological materials into spatial orientations and geometries. Gelatin is a preferred substrate biomaterial because of its very high biocompatibility with living tissues. Indeed, gelatin shows a natural amino acid sequence of Arginine-Glycine-Aspartate (Arg-Gly-Asp) component which is the main amino acid sequence responsible for cell adhesion in extra cellular matrices (ECMs).

In biology, an extracellular matrix (ECM) is a three-dimensional network of extracellular macromolecules, such as collagen, enzymes, and glycoproteins, that provide structural and biochemical support of surrounding cells.

An important parameter for dispensing systems is the dispensing time window, i.e. the time during which a material can be dispensed without showing detrimental viscosity increase upon dispensing, in particular, inside the dispensing head. It is advantageous to have a longer dispensing time window as this gives more flexibility during the dispensing process. However, an important issue nowadays in dispensing technologies and particular in 3D printing is that available printing materials based on gelatin are little or not thermostable and rapidly show an increase in viscosity after the start of the dispensing process, and this increase in viscosity causes the material to block the dispensing head. Dispensing can then only be done for a limited amount of time before the dispensing head is blocked and has to be cleaned or changed before dispensing can be continued. This is not convenient at all to make bigger constructs or to make several smaller constructs. Thus the dispensing time window is with existing gelatin based dispensing material limited. In particular, gelatin, which as mentioned above is very suitable because of its biocompatibility with living tissues, is known to be a high risk material for nozzle clogging. Due to the thermal instability of gelatin, a gelatin solution (prepared for printing typically at concentrations above 7.5%) will rapidly show an increase in viscosity at room temperature (20 to 25 °C), and will block dispensing heads, in particular those having a diameter below 0.64 mm (or 22G). Various solutions exist to overcome this problem. One solution is to dispense the material in a temperature controlled dispensing system. However, such temperature controlled systems are expensive and require great care during operation, which makes the dispensing operation quite complicated. Another solution is to chemically modify the dispensing material or to supplement it with additives. Such currently available bio-printing materials include modified gelatins. Also gelatin can be mixed with other hydrocolloids such as alginates for example. Further, use of only specific raw materials such as fish gelatin may be a solution.

WO 2017216780 A1 relates to a gelatin polymer which is derived from natural sources of cold-adapted marine species, such as salmon gelatin.

There is thus a need to have a dispensing material which is easy to use, e.g. which does not require temperature controlled systems, which has a longer dispensing time window than existing materials, which may be dispensed at higher concentrations than what is currently possible and so on.

The present invention aims to solve at least the above mentioned problems.

### SUMMARY OF THE I NVENTI ON

The present invention is based on the findings of a novel gelatin which has improved properties in particular in the field of dispensing systems, such as 3D printing systems, in particular for the medical field and for food applications, more particularly for the medical field. The gelatin of the present invention allows to have a longer dispensing time window, preferably printing time window. The gelatin of the present invention is thermally stable which is advantageous as it can be dispensed without the need of having complicated temperature controlling systems in place or the need of having complicated formulations with additives or bulking material.

Thus in a first aspect, the present invention relates to a gelatin characterized in that
a. It is soluble in aqueous medium and
b. the first derivative of the FTIR-ATR spectrum remains below zero within the 1440-1355 cm⁻¹ spectral region.

In a second aspect, the present invention relates to the gelatin of the first aspect of the present invention for use in dispensing devices.

In a third aspect, the present invention relates to a gelatin composition comprising the gelatin of the present invention and one or more further ingredients, from the group comprising bulking agents, hydrocolloids and proteins.

In a fourth aspect, the present invention relates to the gelatin composition of the third aspect of the present invention for use in dispensing devices.

In a fifth aspect, the present invention relates to an aqueous gelatin composition comprising from 1 to 30wt% of the gelatin of the present invention or the gelatin composition of the present invention and from 70 to 99wt% of water.

In a sixth aspect, the present invention relates to the aqueous gelatin composition of the fifth aspect of the present invention for use in dispensing devices.

In a seventh aspect, the present invention relates to a construct which comprises the gelatin of the present invention, or the gelatin composition of the present invention or the aqueous gelatin composition of the present invention, and wherein the gelatin is gelled.

In an eight aspect, the present invention relates to a process for making a construct, characterized in that it comprises the steps of
a. providing an aqueous gelatin composition, and
b. feeding the aqueous gelatin composition in a dispensing device, preferably a 3D printer, and
c. operating the dispensing device such as to provide a construct, characterized in that the aqueous gelatin composition is according to the present invention

In a ninth aspect, the present invention relates to the use of the gelatin of the present invention or of the gelatin composition of the present invention, or of the aqueous gelatin composition of the present invention to increase the dispensing time window in dispensing systems, preferably to increase the printability time window in 3D printing systems.

In a tenth aspect, the present invention relates to the use of the gelatin of the present invention, or of the gelatin composition of the present invention or of the aqueous gelatin composition of the present invention to reduce or delay the clogging of dispensing systems during dispensing process, preferably to reduce or delay the clogging of 3D printing systems during 3D printing process.

In an eleventh aspect, the present invention relates to the use of the gelatin of the present invention, or of the gelatin composition of the present invention or of the aqueous gelatin composition of the present invention in the production of scaffolds, engineered tissues, devices or micro-devices suitable for therapeutic purposes, diagnostic purposes, research purposes and the like.

In a twelfth aspect, the present invention relates to the use of the construct of the present invention as a scaffold for tissue engineering, for regenerative medicine, for diagnostic purposes, for drug testing and the like.

### DETAI LED DESCRI PTI ON

### Definitions

As used in the present description, "gelatin" refers to its well-known definition in the art, i.e. it is a linear polymer resulting from partial hydrolysis of collagen. Said partial hydrolysis may be obtained by chemical, enzymatic and/or heat treatment of collagen. In the case of gelatin, hydrolysis of collagen is said to be partial because gelatin keeps some functional properties (such as the ability to form gels) contrary to collagen hydrolysate, which have lost such functional properties of forming gels. Gelatin may be derived from collagen sourced of any suitable type of connective animal tissue, particularly from bones, teeth and hides, preferably collagen is sourced from bones and/or hides. Collagen may be derived from various animal such as pork, beef, sheep, goat, horse, deer, chicken, fish and the like. Preferably however, collagen and thus also gelatin, is derived from pork and/or beef. Standard gelatin requires to be heated in order to dissolve in water. Upon cooling standard gelatin forms a gel, which strength depends on the specific properties of the gelatin such as molecular weight distribution and molecular conformation. Gelatin is mostly characterized by its gel strength, expressed in g Bloom. Further, gelatin is also characterized by a certain viscosity, expressed in mPa.s. Among others, further properties of gelatin are bulk weight or bulk density and particle size.

As used in the present description, "branched gelatin molecules" relates to the intermolecular and intramolecular chemical bonding of gelatin molecules by application of an external factor such as heat for example. The degree of branching of a gelatin composition refers to the percentage of gelatin molecules in said gelatin composition that are branched. Branched gelatin molecules have smaller hydrodynamic radius and, consequently, lower intrinsic viscosity. Apart from that, branched molecules present higher amounts of hydrogen bonds than linear molecules. Less energy is required to break the hydrogen bonds.

As used in the present description, "dispensing systems" or "dispensing devices" refer to apparatus able to deposit a composition, typically as a liquid or paste, onto a surface in the form of dots, lines and any other suitable shapes. Simply stated, said composition is pushed from a reservoir onto a surface through a head, which is typically a nozzle. The head may be driven by pressure, piezoelectric motor or laser-assisted jetting. Depending on the apparatus and on the composition, the head may be of different sizes and different openings, also the need for more or less pressure will depend on the apparatus and composition to be dispensed. Dispensing may be done by extrusion, spraying, jetting and the like. Casting, which is a process comprising filling a mold with a composition to create a specific shape is also understood to be a dispensing process. Dispensing devices are preferably computer controlled systems. A suitable software allows the user to enter a pre-determined shape which is then dispensed on a surface by the dispensing device to provide a construct. Thus a dispensing device may be a 3D printer, an ink jet printer, a single jet dispensing device, and the like. Preferably, the dispensing device is a 3D printing system, i.e. a 3D printer. Preferably, in the present description, dispensing comprises 3D printing.

As used in the present description, "3D printing" refers to a specific dispensing system. 3D printing systems allow to produce tridimensional shaped products, typically by depositing a composition layer by layer until the desired shape is obtained. 3D printing is, as many other dispensing systems, a computer controlled system. Preferably in the present invention, 3D printing systems do not require temperature control during printing.

As used in the present description, "dispensing time window" refers to the time during which the composition to be dispensed can be dispensed without encountering dispensing problems due to, for example, the change in rheology of the composition within the reservoir and/or through the dispensing head of the dispensing system. The dispensing time window starts when dispensing begins and ends when the dispensing head gets clogged by the composition or when the dispensing does no longer happen in a suitable, uniform manner, e.g. undesired lumps are formed, dispensing spots are missed and the like, which is disturbing the desired final shape of the construct.

As used in the present description, "crosslinking" refers to the formation of stable network structure, in particular stable network gel structure, via covalent bonding.

Typically, crosslinking is obtained by the action of an external factor (crosslinking factor) such as exposure to heat, exposure to UV light (photo-initiated chain growth polymerization or crosslinking) and the like, on functionalized molecules. Thus molecules are first functionalized such that crosslinking can happen upon subjection to the right conditions. During functionalization, functional groups are added to the chemical structure of (some of) the molecules by a chemical reaction (chemical functionalization) or by a biochemical reaction (biochemical functionalization) such as an enzymatic reaction for example (enzymatic functionalization). Chemical functionalization may be for example an acrylation reaction, i.e. introducing acrylate substitution groups; a methacrylation reaction, i.e. introducing methacrylate substitution groups, diels -alder, azide-alkyne cycloaddition reaction, thiol-ene chemistry (click chemistry) or a succinylation reaction. Other chemical or biochemical functionalization are not excluded. Molecules may also be previously treated to add reactive groups in their chemical structure in order to give access to new functional chemical groups which will facilitate crosslinking. Alternatively, crosslinking may also be obtained for example by the reaction of certain components present in a composition with specific reactants, for example alginate present in a composition will react and crosslink when exposed to calcium ions, for example when the composition is put in a bath comprising calcium ions. For a composition to be crosslinked, it is sufficient that at least one of its components is crosslinked. For example a composition comprising gelatin and alginate may be crosslinked when it is exposed to calcium ions, in that case only the alginate is in fact crosslinked, however it may be said that the composition is crosslinked.

As used in the present description, "crosslinkable" refers to the ability of a composition to react to an external crosslinking factor causing at least two components of the composition to be crosslinked. A composition can be made crosslinkable by adding functional groups to the chemical structure of (some of) the component(s) of the composition by a chemical or biochemical reaction (see above: functionalization). Alternatively, a composition can be made crosslinkable by adding crosslinking agents (or crosslinking inducers) such as formaldehyde, glutaraldehyde, epoxy compounds and the like. Natural crosslinking agents are for example enzymes (for example transglutaminase) and genipin (an agent extracted from gardenia fruit).

Such natural crosslinking agents can provide lower cytotoxicity levels. Crosslinking agents like e.g. genipin containing more than one reactive group may react with reactive groups on more than one protein molecule and chemically crosslink the gelatin molecules; the crosslink inducers then become part of the crosslinked structure chemically. Enzymes, e.g. transglutaminases or tyrosinases catalyse the chemical reaction between reactive groups on the protein molecule to form chemical bonds and inter- and intra-molecular crosslinks; the enzyme does not become part of the crosslinked network chemically.

The invention will further be illustrated with different embodiments. It is understood that, unless expressly mentioned, all embodiments/aspects may be combined with one another.

In a first aspect, the present invention relates to a gelatin characterized in that
a. it is soluble in aqueous medium and
b. the first derivative of the gelatin's FTIR-ATR spectrum remains below zero within the 1440-1355 cm⁻¹ spectral region.

The gelatin comprises from 95weight% to 100wt%, preferably at least 96wt%, more preferably at least 97wt%, yet even more preferably at least 98wt%, yet even more preferably at least 99wt% of gelatin on dry basis of gelatin (wt% db). The gelatin may indeed comprise some minor amounts of ashes and/or salts which typically result from the processing of collagen into gelatin.

The gelatin of the present invention is soluble in aqueous medium. In this context, solubility in aqueous medium means that when 10g of the gelatin is stirred in 100ml of demi-water at 60°C during 1hour, a clear solution is obtained with no particles visible to the naked eye.

The FTIR-ATR spectrum of a gelatin sample is obtained with a resolution of 4 cm⁻¹, accumulating 16 scans per spectrum. Spectral measurements are performed within the mid-infrared range of 4500-600 cm⁻¹. Measurement is performed with a diamond triple-bounce ATR accessory (Bruker, Tensor 27). Background air spectrum is collected before each sample measurement. A sample is directly compressed on attenuated total reflectance (ATR) crystal prior to analysis without any preliminary preparation. After measurement of a gelatin sample, ethanol and distilled water are used to clean the diamond ATR crystal, which has to dry before performing a next measurement. Between each sample measurement a blank measurement is done (i.e. measurement without presence of sample). The spectra is depicted in transmittance mode.

OriginPro8.5 is used to calculate the first derivative of the spectra within the 1440 - 1355 cm-1 wavenumber range. The signal is smoothed by applying a quadratic Savitzky-Golay filter with 5-points.

The gelatin of the present invention may be obtained by a process of flash hydrolysis followed by rapid evaporation of water (see example 1 for instance).

Preferably the gelatin is further characterized in that it shows a shift of the peak in the region of the Amide I band (C=O stretch) (1660-1600 cm⁻¹) of about 1 cm⁻¹, preferably of about 0.5 cm⁻¹ towards the high ranges of wavenumber, compared to the gelatin starting material.

More preferably, the gelatin is further characterized in that it shows a shift of the peak in the region of the C-N-H bending band (1565-1500 cm⁻¹) of about 9 cm⁻¹, preferably of about 7 cm⁻¹, more preferably about 5 cm⁻¹ towards the lower ranges of wavenumber, compared to the gelatin starting material.

Preferably, the gelatin is in powder form, particle size may vary but is preferably 70 to 8 mesh, more preferably 60 to 15 mesh, even more preferably 60 to 20 mesh, yet even more preferably 50 to 30 mesh, yet even more preferably 40 to 30 mesh.

Preferably, the gelatin has a gel strength of above 50g Bloom, preferably from 50g to 360g Bloom, preferably 80g to 350g Bloom, more preferably 100g to 350g Bloom, yet even more preferably 120g to 340g Bloom, yet even more preferably 150g to 340g Bloom, yet even more preferably 180g to 330g Bloom, yet even more preferably 200g to 330g Bloom, yet even more preferably 250g to 330g Bloom, yet even more preferably 270g to 320g Bloom, yet even more preferably 280g to 320g Bloom.

In one embodiment, the gelatin of the present invention is substantially not functionalized. Substantially not functionalized means that the degree of functionalization, in particular the degree of chemical functionalization is less than 5%, more preferably from 3% to 0%, yet even more preferably from 1% to 0%, most more preferably from 0.1% to 0%. Functionalization is as defined above.

In another embodiment, the gelatin of the present invention is functionalized. Functionalization is as defined above. It is an advantage of the present gelatin that due to its high amounts of gelatin, a functionalization reaction is easier to perform, compared to other dispensing material where the composition renders a functionalization reaction more difficult to perform, or makes it more difficult to find the right reaction conditions. Preferably the degree of functionalization is from 30% to 95%, more preferably from 35 to 90%, yet even more preferably from 40 to 85%, yet even more preferably from 45 to 80%, yet even more preferably from 50 to 75%, yet even more preferably from 55 to 70%, yet even more preferably from 60 to 65%. The levels of functionalization will depend on the application. Higher degrees of functionalization is indicated for applications that require more stable structure in physiological conditions of pH and temperature. Preferably the gelatin is chemically functionalized, more preferably the gelatin is acrylated or methacrylated, more preferably the gelatin is methacrylated.

In a further embodiment, the gelatin comprises functionalized gelatin of the present invention and substantially non functionalized gelatin of the present invention. Thus from 1 to 99wt%, preferably from 5 to 90wt%, more preferably from 10 to 80wt%, even more preferably from 20 to 60wt%, yet even more preferably from 30 to 50wt% yet even more preferably from 40 to 50wt% of the gelatin is functionalized and from 1 to 99wt%, preferably from 5 to 90wt%, more preferably from 10 to 80wt%, even more preferably from 20 to 60wt%, yet even more preferably from 30 to 50wt% yet even more preferably from 40 to 50wt% is substantially not functionalized. The degree of functionalization is as described here above.

During methacrylation, methacrylate groups are introduced on the gelatin molecules through a well-known process: typically gelatin is dissolved in a solvent, typically phosphate buffer saline (PBS) or dimethyl sulfoxide solvent (DMSO), methacrylic anhydride is then directly added to said gelatin solution, the methacrylation reaction is conducted at about 50°C. The degree of methacylation can be controlled by the amount of methacrylic anhydride added. The gelatin of the present invention may be methacrylated and may have a degree of methacrylation as mentioned above.

During acrylation, acrylate groups are introduced on the gelatin molecules through a well-known process. Reference is made for example to Billiet, I.T. (2014) "Gelatin Functionalizations for Cell Embedding in 3D Geometries Using Rapid Prototyping Technology".

Preferably further, the gelatin of the present invention comprises less than 100 Endotoxin Units (EU), more preferably less than 80 EU, even more preferably less than 60 EU, yet even more preferably less than 40 EU, yet even more preferably less than 30 EU, yet even more preferably less than 10 EU per gram of gelatin, expressed on as is basis. Endotoxins may be removed by clearance filters for endotoxins removal such as charged filters. Such charged filters are available and can be for example Mustang E® membrane and Mustang Q® membrane (from Pall), Sartobind® filter (from Sartorious). The LAL test (acronym for Limulus Amebocyte Lysate) is a test for the determination of bacterial endotoxins, which uses an Amebocyte Lysate of the Limulus crab.

Further preferably the gelatin of the present invention is purified by using one or more of the following techniques: ultrafiltration, diafiltration, filtration with depth filters. These filtration techniques are useful to (partially) remove for example undesired toxins, bacteria, product fractions and the like.

Preferably the gelatin of the present invention comprises less than 5wt%, more preferably less than 4wt%, even more preferably less than 3wt%, yet even more preferably less than 2wt%, yet even more preferably less than 1wt%, yet even more preferably less than 0.5wt%, yet even more preferably less than 0.1wt% of additives, on dry basis of the gelatin. More preferably, the gelatin of the present invention comprises substantially no additives. Additives may be rheological enhancers, flowability enhancers and the like, such as for example glycols, starches, alginates and the like. It is an advantage of the present gelatin to comprises such low amounts of additives in the ranges mentioned above, in particularly to comprise substantially no additives, in particular when the gelatin is used to produce scaffolds for use as ECM with living cells as the additives have a negative effect on cell survival rates. Many current material for producing scaffolds for use as ECM nowadays comprise additives in order to allow the materials to be dispensed, in particular to be 3D printed into said scaffolds.

The gelatin of the present invention is alternatively characterized in that when dispensed
a. as a 10wt% solution in water, and
b. under pressure from 20 to 60kPa, and
c. at room temperature, and
d. through a nozzle of from 0.01 to 3mm,
   said solution keeps a substantially consistent flow behavior for at least 15 minutes. Dispensing is to be done at most 3hours after preparation of said solution. The solution is prepared by dissolving gelatin at 50°C for 30minutes
   after which the solution may be returned to room temperature (20 to 25°C).

Preferably, said solution keeps a substantially consistent flow behavior for a time of from above 15 minutes to 3 hours, more preferably from 20 minutes to 2 hours and 30 minutes, even more preferably from 30 minutes to 2 hours, yet even more preferably from 40 minutes to 2 hours, yet even more preferably from 40 minutes to 2 hours, yet even more preferably from 50 minutes to 2 hours, yet even more preferably from 1 hour to 2 hours.

In a second aspect, the present invention relates to the gelatin of the first aspect of the present invention for use in dispensing devices, preferably for use in a 3D printer.

It has been found that said gelatin is particularly suitable for dispensing, in particular for use as a bio-ink in 3D printing. The present invention also relates to the gelatin of the first aspect of the present invention for use in the construction of scaffold for medical applications, preferably for tissue engineering, for regenerative medicine, for implants, for medical devices, for drug discovery and drug testing, or for cosmetic applications, preferably a scaffold for testing of cosmetic products, and the like. The gelatin can be dispensed without the need of having a temperature controlled dispensing system and without the need of having other ingredients added, or additives added or other solutions that are typically used in the art to be able to dispense gelatin. Preferably dispensing is done under pressure, more preferably at room temperature. Dispensing, and particularly printing, is done by putting the material into an aqueous solution. The solvent may be a phosphate buffer, bovine serum albumin (BSA), water, preferably the solvent is a phosphate buffer or BSA, more preferably a phosphate buffer. Advantageously, the gelatin is put in solution at a temperature of from 30 to 60°C, more preferably from 40 to 55°C, yet even more preferably from 40 to 50°C and allowed to cool down to room temperature before dispensing. It is preferable to start the dispensing process before a period of maximum 3 hours after cool down.

Further advantage of the present gelatin is that it can be dispensed as a highly concentrated aqueous solution, i.e. aqueous solution containing up to 30wt% of the gelatin of the present invention, preferably the gelatin of the present invention is used in dispensing devices as a solution comprising from 1 to 30wt%, more preferably from 5 to 25wt%, even more preferably from 10 to 20wt%, yet even more preferably from 10 to 15wt% of the gelatin of the present invention.

In a third aspect, the present invention relates to a gelatin composition comprising the gelatin of the first aspect of the present invention and further ingredients. Preferably the gelatin composition is in powder form.

The gelatin composition comprises one or more of bulking agents, hydrocolloids, proteins, anticaking agents and the like. Preferably the gelatin composition of the present invention comprises from 40wt% to 95wt%, more preferably from 45wt% to 95wt%, even more preferably from 50wt% to 95wt%, yet even more preferably from 55wt% to 95wt%, yet even more preferably from 60wt% to 95wt%, yet even more preferably from 65wt% to 95wt%, yet even more preferably from 70wt% to 95wt%, yet even more preferably from 75wt% to 95wt%, yet even more preferably from 80wt% to 95wt%, yet even more preferably from 85wt% to 95wt% of the gelatin of the present invention, on dry basis of the gelatin composition.

Bulking agents are well-known in the art and refer to material which do not possess any functionality, inert products, such as sugars, mono-, di- and tri-saccharides, maltodextrins, fibers, protein hydrolysates (such as collagen hydrolysates for example) and the like.

Hydrocolloids may be one or more of alginate, pectin, carrageenan, gellan, agar, starch, xanthan gum, locust bean gum, guar gum, gum karaya, gum tragacanth, gum Arabic, cellulose derivatives, chitosan and the like. Preferably the hydrocolloids are alginate or carrageenan, more preferably alginate.

Proteins may be one or more of keratin, elastin, fibroin, thrombin, albumin, and the like. Protein may also be regular gelatin.

In an embodiment, the gelatin composition comprises functionalized gelatin of the present invention and regular gelatin (i.e. not according to the present invention) which is not functionalized. The gelatin composition preferably comprises from 1 to 99wt%, preferably from 5 to 90wt%, more preferably from 10 to 80wt%, even more preferably from 20 to 60wt%, yet even more preferably from 30 to 50wt% yet even more preferably from 40 to 50wt% of functionalized gelatin of the present invention and from 1 to 99wt%, preferably from 5 to 90wt%, more preferably from 10 to 80wt%, even more preferably from 20 to 60wt%, yet even more preferably from 30 to 50wt% yet even more preferably from 40 to 50wt% of regular gelatin, non-functionalized. The degree of functionalization is as described above.

Further preferably the gelatin composition of the present invention has been purified by using one or more of the following techniques: ultrafiltration, diafiltration, filtration with depth filters. These filtration techniques are useful to remove or at least substantially lower the amount of bacteria, endotoxins, fats, non-collagen proteins, fibres and the like.

In a fourth aspect the present invention relates to the gelatin composition of the third aspect of the present invention for use in dispensing devices, preferably in 3D printer. It has been found that said gelatin composition is particularly suitable for dispensing, in particular for use as a bio-ink in 3D printing. The present invention also relates to the gelatin composition of the third aspect of the present invention for use in the construction of scaffold for medical applications, preferably for tissue engineering, for regenerative medicine, for implants, for drug discovery and drug testing, or for cosmetic applications, preferably a scaffold for testing of cosmetic products and the like, or for food applications, such as for example personalized meals, reconstituted meals and the like.

The gelatin composition can be dispensed when put into solution without the need of having a temperature controlled dispensing system and without the need of having additives added or other solutions that are typically used in the art to be able to dispense gelatin composition. Preferably dispensing is done under pressure, more preferably at room temperature. Dispensing, and particularly printing, is done with the gelatin composition put into an aqueous solution. The solvent may be a phosphate buffer, BSA, water, preferably it is a phosphate buffer or BSA, more preferably a phosphate buffer. Advantageously, the gelatin composition is put in solution at a temperature of from 30 to 60°C, more preferably from 40 to 55°C, yet even more preferably from 40 to 50°C and allowed to cool down to room temperature before dispensing. It is preferable to start the dispensing process maximum before a period of 3 hours after cool down.

A further advantage of the present gelatin composition is that it can be dispensed as a highly concentrated aqueous solution, i.e. aqueous solution containing up to 30wt% of the gelatin composition, preferably the present gelatin composition is used in dispensing devices as a solution comprising from 1 to 30wt%, more preferably from 5 to 25wt%, even more preferably from 10 to 20wt%, yet even more preferably from 10 to 15wt% of the gelatin composition.

In a fifth aspect, the present invention relates to an aqueous gelatin composition comprising from 1 to 30wt% of the gelatin of the first or second aspect of the present invention or from 1 to 30wt% of the gelatin composition of the third or fourth aspect of the present invention and from 70 to 99wt% of an aqueous medium.

The aqueous medium may be a phosphate buffer, bovine serum albumin (BSA), water, preferably the solvent is a phosphate buffer or BSA, more preferably a phosphate buffer.

Preferably, the aqueous gelatin composition of the present invention comprises various components, preferably cell growth components, i.e. components which have a beneficial effect on the growth of living plant, human or animal cells. Such components may be cytokines (Adiponectin Human) cell growth promoters or growth factors, such as (EGF, FGF, NGF, PDGF, VEGF, IGF, GMCSF, GCSF, TGF, Erythropieitn, TPO, BMP, HGF, GDF, Neurotrophins, MSF, SGF, GDF); nutrients, such as glucose, yeast extract; salts, such as potassium chloride, sodium chloride and the like; proteins, such as thrombin transferrin, albumin, peptones and the like.

Preferably, the aqueous gelatin composition of the present invention further comprises living organisms, preferably human, animal and/or plant cells. Said living organisms may be encapsulated or non-encapsulated.

Advantageously, the aqueous gelatin composition of the present invention is prepared by bringing all components in solution at a temperature of from 30 to 60°C, preferably from 40 to 55°C, yet even more preferably from 40 to 50°C and letting the aqueous composition cool down to room temperature before further use.

Further preferably the aqueous gelatin composition of the present invention has been purified by using one or more of the following techniques: ultrafiltration, diafiltration, filtration with depth filters. These filtration techniques are useful to remove or at least substantially lower the amount of bacteria, endotoxins (removal of e.g. from 10 to 90% of endotoxins), fats, non-collagen proteins, fibres and the like.

In a sixth aspect, the present invention relates to the aqueous gelatin composition of the present invention for use in dispensing devices, preferably in 3D printer. It has been found that said aqueous gelatin composition is particularly suitable for dispensing, in particular for use as a bio-ink in 3D printing. The present invention also relates to the aqueous gelatin composition of the present invention for use in the construction of scaffold for medical applications, preferably for tissue engineering, for regenerative medicine, for implants, for drug discovery and drug testing, or for cosmetic applications, preferably a scaffold for testing of cosmetic products, and the like. Also the present aqueous is very suitable for dispensing, preferably 3D printing, or food applications.

The aqueous gelatin composition can be dispensed without the need of having a temperature controlled dispensing system and without the need of having additives added or other solutions that are typically used in the art to be able to dispense aqueous gelatin compositions. Preferably dispensing is done under pressure, more preferably at room temperature.

Advantageously, the aqueous gelatin composition is heated to a temperature of from 30 to 60°C, more preferably from 40 to 55°C, yet even more preferably from 40 to 50°C and allowed to cool down before dispensing. It is not desired to keep the cooled aqueous gelatin composition for a period of time of more than 3hours at room temperature before starting the dispensing process.

In a seventh aspect, the present invention relates to a construct, comprising the gelatin of the present invention, the gelatin composition of the present invention and/or the aqueous gelatin composition of the preset invention, wherein the gelatin is gelled.

As mentioned above, the gelatin of the present invention may or may not be functionalized. When functionalized, the construct is crosslinkable, and crosslinking may be done by application of the right crosslinking conditions (such as applying a photoinitiator for example). When crosslinking reaction has been performed, it may be said that the construct is crosslinked. Crosslinked constructs, especially comprising gelatin which is a natural ingredient that melts at body temperature, are very stable and can be introduced in the body or used at temperatures above the melting temperature of gelatin, without the fear that the construct would melt away or show deformations.

Preferably crosslinking takes place immediately after dispensing (filament deposition) to ensure self-supporting properties of the 3D-construct, to avoid collapsing of the 3D construct after printing.

The construct may further comprise one or more of other components such as carbohydrates, salts, living cells, cell growth promoters and enzymes.

The construct may comprise the gelatin, the gelatin composition and/or the aqueous gelatin composition of the present invention ('gelatin comprising compositions') and other further components due to for example dispensing the construct with a multiple headed nozzle, where one or more nozzles dispensing the gelatin comprising composition and the other one or more nozzles dispensing another type of suitable composition which can be dispensed and is preferably biocompatible, such as for example a composition comprising hydrocolloids, for example alginate; proteins; carbohydrates and the like.

In an eight aspect, the present invention relates to a process for making a construct, characterized in that the process comprises the steps of
a. providing an aqueous gelatin composition, and
b. feeding the aqueous gelatin composition in a dispensing device, preferably a 3D printer, and
c. operating the dispensing device such as to provide a construct, characterized in that the aqueous gelatin composition is according the present invention.

Operating the dispensing device may be done at a controlled temperature such as at a temperature of from above 25 to 40°C, however advantageously with the present aqueous gelatin composition, dispensing is done without controlled temperature system, at room temperature (20 to 25°C).

Preferably the construct is made by dispensing layer above layer or dot above dot of the aqueous gelatin composition. If needed the construct may also be a single layer or dot of the aqueous gelatin composition. In fact, any suitable construct may be printed, in accordance with the possibilities of the used 3D printer software.

Once the construct is made, it may be crosslinked, provided either that the aqueous gelatin composition was functionalized to be crosslinked or provided that enzymes have been added which can cause crosslinking. Preferably crosslinking takes place right after dispensing (filament deposition), i.e. the time depending on the viscoelastic properties of the dispended material, to ensure self-supporting properties of the 3D-construct, thus to avoid collapsing of the 3D construct after printing.

It is also possible to start the crosslinking reaction whilst dispensing, either directly from the start of the dispensing step or at any suitable time after the start of the dispensing step.

Typically, accurate time control during dispensing process is important when crosslinking agents are present in the aqueous gelatin composition because the crosslinking reaction may cause detrimental viscosity increase inside the nozzle while dispensing. In this case, gelatin thermostability of the gelatin of the present invention is advantageous in order to prevent the combined effect of physical gel formation together with increments of viscosity caused by (chemical) crosslinking, making it possible to have a more flexible time control during dispensing process.

The present invention further relates to the use of the gelatin of the present invention, the gelatin composition of the present invention or the aqueous gelatin composition of the present invention to increase the dispensing time window in dispensing systems, preferably to increase the printability time window in 3D printing systems.

This effect is due to the fact that said gelatin, gelatin composition and aqueous gelatin compositions show little to no viscosity increase during printing, also at room temperature, for a period of time of up to 3 hours, preferably of above 15 minutes to 3 hours, more preferably from 20 minutes to 2 hours and 30 minutes, even more preferably from 30 minutes to 2 hours, yet even more preferably from 40 minutes to 1 hour and 30 minutes, yet even more preferably from 40 minutes to 1 hour. In comparison, previous compositions comprising gelatin typically only allowed a dispensing time window of about 15 minutes or less. As a results of this little to no viscosity increase, clogging of the dispensing head or nozzle, preferably printing nozzle does not happen until after 3 hours of dispensing, preferably printing, preferably not until after a period of above 15 minutes to 3 hours, more preferably from 20 minutes to 2 hours and 30 minutes, even more preferably from 30 minutes to 2 hours, yet even more preferably from 40 minutes to 2 hours, yet even more preferably from 40 minutes to 2 hours, yet even more preferably from 50 minutes to 2 hours, yet even more preferably from 1 hour to 2 hours.

Further the present invention relates to the use of the gelatin, gelatin composition and aqueous gelatin compositions of the present invention in the preparation of a scaffold for medical applications, preferably for tissue engineering, for regenerative medicine, for implants, for drug discovery and drug testing, or for cosmetic applications, preferably a scaffold for testing of cosmetic products, and the like. Also the present invention relates to the use of the gelatin, gelatin composition and aqueous gelatin compositions of the present invention in the preparation of food applications such as customized meals, reconstituted foods, bakery applications and the like.

Further, the present invention relates to the use of the construct of the present invention in the preparation of a scaffold for medical applications, preferably for tissue engineering, for regenerative medicine, for implants, for drug discovery and drug testing, or for cosmetic applications, preferably a scaffold for testing of cosmetic products, and the like.

The present invention will be further illustrated in the non-limiting examples.

### EXAMPLES

### Example 1: production of gelatin according to the present invention

A solution of alkaline treated porcine gelatin of high bloom (300g) (T=60°C) at a concentration of 35wt% is submitted to flash hydrolysis by heating to achieve a product temperature of 120°C for 60 seconds. The heated gelatin is then submitted to evaporation by roll-coating the heated gelatin solution on the surface of a rotating drum heated by steam at 4.5 bar. The adhering thin layer of product on the surface of the drum is rapidly dried and scratched off by the action of a sharp knife. The gelatin so produced is soluble in water.

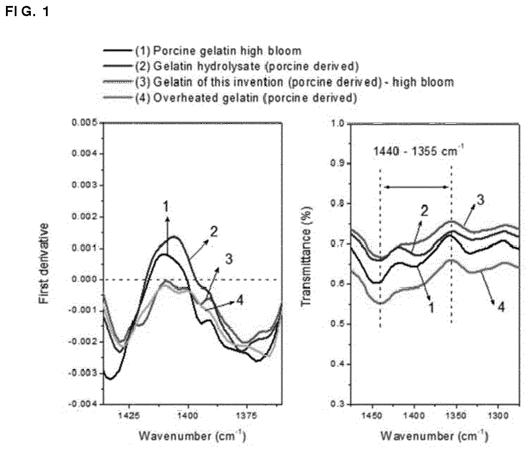

The FTIR-ATR spectrum of the gelatin so produced is measured. The first derivative and the transmittance is depicted in figure 1 (3).

FTIR-ATR spectrum of following material are also depicted in figure 1:
- Regular porcine gelatin of high bloom (300g) (1)
- Porcine derived gelatin hydrolysate (2)
- Overheated porcine derived gelatin (4). Said gelatin has been exposed to 150°C for 2 hours and is not soluble in water.

### Figure 1: FTIR-ATR spectrum of several gelatin samples, data is recorded in transmittance mode

As depicted in figure 1, the first derivative of the spectrum of the gelatin of the present invention remains below zero within the 1440-1355 cm⁻¹ spectral region. Gelatin (1) and the hydrolysed gelatin (2) depict within that specific spectral region two zero-crossing values, i.e. the derivative crosses zero twice within that region.

### Example 2: preparation of a construct with gelatin according to the invention and with regular gelatin

Comparative experiment demonstrating the printing behavior of gelatin from example 1 in comparison with regular gelatin was conducted. Both gelatins are derived from porcine source and present bloom higher than 280g, about 300g. On a Cellink Inkredible 3D printer, constructs are is prepared with the gelatin according to example 1 and with the regular gelatin.

The gelatin of example 1 and the regular gelatin are each dispersed in a glass vial containing PBS (phosphate buffer solution, pH7.4) to produce a solution at a concentration of 10w/w%. The dispersion of the powder in solution is performed using a vortex mixer (3000 rpm). The vials containing 10mL of solution are then placed in a water bath at 60 °C for 1 hour. While warm, the solutions are then each poured into a 3mL syringe from the 3D printer.

The 2 syringes are placed on the bench and allowed to cool at ambient temperature conditions (about 20°C).

The syringe containing the gelatin according to example 1 is placed in the printhead 1 (PH1) and the syringe containing the regular gelatin is placed in the printhead 2 (PH2). Printing of 6 lattice structures was initiated simultaneously by PH1 and PH2.

The printing settings applied on the Slic3r software for printing of a lattice structure are adjusted as follows:

| Nozzle size | Printing speed | Infill | Pressure PH1 | Pressure PH2 | Temperature |
|---|---|---|---|---|---|
| 25G (0.3 mm) | 150 mm min-1 | 12% | 85-100kPa | 130-140kPa | 23-25 °C |

The gelatin according to example 1 requires extrusion pressure of 85-100kPa while the regular gelatin requires a pressure of 130-140kPa in order to have filament formation.

| 12 layer count lattice structure | Time required to print (mins) | Gelatin from example 1 | Regular gelatin |
|---|---|---|---|
| Lattice 1 | 19m:15s | Complete | Complete |
| Lattice 2 | 18m:03s | Complete | Complete |
| Lattice 3 | 18m:06s | Complete | Not initiated due to nozzle blockage |
| Lattice 4 | 19m:08s | Complete | Not initiated due to nozzle blockage |
| Lattice 5 | 17m:54s | Complete | Not initiated due to nozzle blockage |
| Lattice 6 | 18m:01s | Complete | Not initiated due to nozzle blockage |

With the regular gelatin, printing of a third lattice construct is not possible due to complete blockage of the nozzle. With the gelatin according to example 1, it is possible to print up to 6 lattice constructs, printing can be done up to 105 minutes without any blockage of the nozzle and uniform filament production, the flow behavior remains uniform during the entire printing operation.

## Claims

1. A gelatin **characterized in that**
a. it is soluble in aqueous medium and
b. the first derivative of the gelatin's FTIR-ATR spectrum remains below zero within the 1440-1355 cm⁻¹ spectral region.

2. The gelatin of claim 1 further **characterized in that** it is substantially not functionalized.

3. The gelatin according to claims 1 or 2, further **characterized in that** it is functionalized, preferably chemically functionalized.

4. The gelatin of any one of the previous claims further **characterized in that** it comprises less than 5wt% additives, such as rheological enhancers, flowability enhancers and the like.

5. The gelatin of any one of the previous claims further **characterized in that** it comprises less than 100 Endotoxin Units (EU) per gram of gelatin composition.

6. The gelatin of any one of claims 1 to 5 for use in dispensing devices, preferably in a 3D printing system, preferably wherein dispensing is done under pressure, preferably further at room temperature.

7. The gelatin of according to claim 6, for use in the construction of a scaffold for medical applications, preferably for tissue engineering, for regenerative medicine, for implants, for drug discovery and drug testing, or for cosmetic applications, preferably a scaffold for testing of cosmetic products, and the like.

8. A gelatin composition comprising the gelatin of any one of claims 1 to 7 and one or more further ingredient, chosen from the group comprising bulking agents, hydrocolloids and proteins.

9. The gelatin composition of claim 8 further **characterized in that** it comprises from 55 to 90wt% of the gelatin and from 10 to 45wt% of the one or more further ingredient.

10. An aqueous gelatin composition comprising from 1 to 30wt% of the gelatin of any one of claims 1 to 7 or the gelatin composition of claims 8 or 9 and from 70 to 99wt% of an aqueous medium, preferably a phosphate buffer or bovine serum albumin, more preferably a phosphate buffer.

11. The aqueous gelatin composition of claim 10 further **characterized in that** it comprise encapsulated or non-encapsulated living organisms, preferably living human, animal and/or plant cells, and/or further **characterized in that** it comprises cell growth components, such as cell growth promoters, nutrients, salts, proteins and the like.

12. The aqueous gelatin composition of any one of claims 10 to 11 for use in dispensing devices, preferably in 3D printing system, preferably wherein dispensing is done under pressure, preferably further at room temperature.

13. The aqueous gelatin composition of any one of claims 10 to 12 for use in the construction of a scaffold for medical applications, preferably for tissue engineering, for regenerative medicine, for implants, for drug discovery and drug testing, or for cosmetic applications, preferably a scaffold for testing of cosmetic products, and the like.

14. A construct comprising the gelatin according to any one of claims 1 to 7 or the gelatin composition according to any one of claims 8 to 9 characterized the gelatin is gelled, preferably further **characterized in that** the construct is cross-linked.

15. A process for making a construct, **characterized in that** it comprises the steps of
a. providing an aqueous gelatin composition, and
b. feeding the aqueous gelatin composition of step a. in a dispensing device, preferably a 3D printer, and
c. operating the dispensing device such as to provide a construct, **characterized in that** the aqueous gelatin composition is according to any one of claims 10 to 13.
